# EUROPEAN PATENT APPLICATION

(11) **EP 3 437 621 A1**
(43) Date of publication of application: **06.02.2019**
(21) Application number: 17775173.2
(22) Date of filing: 29.03.2017
(51) Int. Cl.: A61J 1/16, A61J 1/06, A61M 5/00, B65B 55/04

(54) **SYRINGE RETAINING MEMBER, SYRINGE PACKAGE BODY, AND METHOD FOR ASSEMBLY OF SYRINGE PACKAGE BODY**

(30) Priority: 31.03.2016 JP 2016071849
(71) Applicant: Terumo Kabushiki Kaisha, Tokyo 151-0072 (JP)
(72) Inventor: OKIHARA, Hitoshi, Fujinomiya-shi Shizuoka 418-0004 (JP)
(74) Representative: Casalonga
(86) International application number: PCT/JP2017/012796
(87) International publication number: WO 2017/170636

(57) **Abstract**

Provided is a syringe retaining member, comprising a base plate part, a plurality of retaining cylinder parts, and reinforcing ribs. The reinforcing ribs protrude from the upper surface of the base plate part, and connect the adjacent retaining cylinder parts. Among the plurality of retaining cylinder parts, two or more of the reinforcing ribs are disposed between a prescribed pair of the adjacent retaining cylinder parts.

## Description

### Technical Field

The present invention relates to a syringe holding member that holds a plurality of syringes, a syringe package body including the syringe holding member, and a method for assembling the syringe package body.

### Background Art

Conventionally, there is a widely used syringe storage container capable of holding a plurality of syringes in an upright state for conveying or storing a syringe before a medicinal solution or the like is filled. Such a syringe storage container is constituted with a box-shaped container main body having an open upper surface and a syringe holding member capable of holding a plurality of syringes in an upright state. A shelf-like portion for supporting the syringe holding member is provided inside the container main body.

The syringe holding member is constituted with a plate member including a plurality of through holes. The syringe is inserted into the through hole and a flange portion provided at a syringe end portion is set to be caught on the through hole, whereby the syringe is held by the syringe holding member. With the use of this type of syringe storage container, it is possible to hold a plurality of syringes in an upright state, with the filling port of the medicinal solution facing upward.

In addition, sterilizing operation using high temperature steam or gas such as high pressure steam sterilization (autoclave) or ethylene oxide gas (EOG) sterilization, or medicinal solution filling operation are performed with the plurality of syringes held by the syringe holding member. Then, a gasket for pushing out the medicinal solution is capped to the syringe filled with the medicinal solution, in a state where the syringe is held by the syringe holding member.

A storage container described in Patent Literature 1 is known as an exemplary storage container as described above. A medical container described in Patent Literature 1 includes a box-shaped container main body with an open upper surface, a plate-like syringe holding member installed in the container main body, and a plurality of cylindrical holding portions formed in the syringe holding member. In addition, this medical container connects adjacent holding portions with each other with one connecting rib for one connection in order to increase the strength of the entire syringe holding member.

### Citation List

### Patent Literature

Patent Literature 1: WO 2008/107961

### Summary of Invention

### Technical Problem

This, however, includes a disadvantage that when the syringe holding member holding a large number of syringes by a plurality of holding portions is sterilized by high temperature steam by high pressure steam sterilization (autoclave), the syringe holding member might bend and deform due to the heat of the steam and the load of the syringe even with the space between the adjacent holding portions being connected by one connecting rib. Deformation of the syringe holding member might change an interval between the plurality of adjacent syringes being installed, affecting operation of filling medicinal solution or gasket capping, that is, hindering high accuracy operation of filling medicinal solution or gasket capping.

In view of the above problem, the present invention aims to provide a syringe holding member, a syringe package body, and a method for assembling a syringe package body capable of increasing the strength of a base plate portion and suppressing deformation at the time of high pressure steam sterilization.

### Solution to Problem

In order to solve the above problem and achieve the object of the present invention, a syringe holding member according to the present invention includes a base plate portion, a plurality of holding tube portions, and a plurality of reinforcement ribs. The base plate portion is formed in a substantially rectangular flat plate shape including a long side and a short side. The plurality of holding tube portions protrudes from an upper surface of the base plate portion and includes a cylindrical hole into which a syringe is inserted and penetrating the base plate portion. The reinforcement rib protrudes from the upper surface of the base plate portion and connects each of the plurality of holding tube portions to each of the other plurality of adjacent holding tube portions. In addition, two or more reinforcement ribs are provided between two adjacent predetermined holding tube portions among the plurality of holding tube portions.

The syringe package body of the present invention includes the above-described syringe holding member, a plurality of syringes each held by each of a plurality of holding tube portions of the syringe holding member, a container main body, and a sheet-like sealing member. The container main body includes: a peripheral wall portion including an upper end portion and a lower end portion; a bottom portion continuous with the lower end portion of the peripheral wall portion and surrounded by the lower end portion; an opening portion surrounded by the upper end portion; a storage space formed with the peripheral wall portion, the bottom portion, and the opening portion; and a mounting shelf formed in the peripheral wall portion. The sealing member seals the opening portion of the container main body and is formed of a material including gas permeability and bacteria impermeability.

The syringe holding member is mounted on the mounting shelf and stored in the storage space.

Furthermore, a method for assembling a syringe package body of the present invention includes the steps (1) to (5) as follows, involving the above-described syringe holding member, the plurality of syringes, the container main body, and the sealing member.
(1) Step of preparing the syringe holding member described above.
(2) Step of preparing the container main body described above.
(3) Step of inserting each of a plurality of syringes into each of a plurality of holding tube portions of the syringe holding member.
(4) Step of mounting the syringe holding member holding the plurality of syringes on the mounting shelf of the container main body to be stored in the storage space.
(5) Step of applying high pressure steam sterilization on the plurality of syringes, the syringe holding member, the container main body, and the sealing member.

### Advantageous Effects of Invention

With the syringe holding member, the syringe package body, and the method for assembling the syringe package body according to the present invention, it is possible to increase the strength of the base plate portion and to suppress deformation of the syringe holding member at the time of sterilization involving a heat load, high pressure steam sterilization in particular.

### Brief Description of Drawings

Fig. 1 is a perspective view illustrating a syringe package body according to a first exemplary embodiment of the present invention.
Fig. 2 is a front perspective view illustrating a syringe holding member according to a first exemplary embodiment of the present invention.
Fig. 3 is a front view illustrating the syringe holding member according to the first exemplary embodiment of the present invention.
Fig. 4 is a rear view illustrating the syringe holding member according to the first exemplary embodiment of the present invention.
Fig. 5 is a plan view illustrating the syringe holding member according to the first exemplary embodiment of the present invention.
Fig. 6 is a bottom view illustrating the syringe holding member according to the first exemplary embodiment of the present invention.
Fig. 7 is a left side view illustrating the syringe holding member according to the first exemplary embodiment of the present invention.
Fig. 8 is a right side view illustrating the syringe holding member according to the first exemplary embodiment of the present invention.
Fig. 9 is a cross sectional view illustrating a state in which a syringe is stored in a syringe storage container according to the first exemplary embodiment of the present invention.
Fig. 10 is a front perspective view illustrating a syringe holding member according to a second exemplary embodiment of the present invention.
Fig. 11 is a front view illustrating the syringe holding member according to the second embodiment of the present invention.
Fig. 12 is a rear view illustrating the syringe holding member according to the second embodiment of the present invention.
Fig. 13 is a plan view illustrating the syringe holding member according to the second embodiment of the present invention.
Fig. 14 is a bottom view illustrating the syringe holding member according to the second embodiment of the present invention.
Fig. 15 is a left side view illustrating the syringe holding member according to the second embodiment of the present invention.
Fig. 16 is a right side view illustrating the syringe holding member according to the second embodiment of the present invention.
Fig. 17 is a front perspective view illustrating a syringe holding member according to a third exemplary embodiment of the present invention.
Fig. 18 is a front view illustrating the syringe holding member according to the third exemplary embodiment of the present invention.
Fig. 19 is a rear view illustrating the syringe holding member according to the third exemplary embodiment of the present invention.
Fig. 20 is a plan view illustrating the syringe holding member according to the third exemplary embodiment of the present invention.
Fig. 21 is a bottom view illustrating the syringe holding member according to the third exemplary embodiment of the present invention.
Fig. 22 is a left side view illustrating the syringe holding member according to the third exemplary embodiment of the present invention.
Fig. 23 is a right side view illustrating the syringe holding member according to the third exemplary embodiment of the present invention.
Fig. 24 is a front view illustrating a syringe holding member according to a fourth exemplary embodiment of the present invention.
Fig. 25 is a front view illustrating a syringe holding member according to a fifth exemplary embodiment of the present invention.
Fig. 26 is a cross sectional view illustrating a syringe package body according to a sixth exemplary embodiment of the present invention.
Fig. 27 is a plan view illustrating a sealing member according to the sixth exemplary embodiment of the present invention.
Fig. 28 is a cross sectional view illustrating a syringe package body according to a seventh exemplary embodiment of the present invention.
Fig. 29 is a plan view illustrating a sealing member and an inner sheet according to the seventh exemplary embodiment of the present invention.

### Description of Embodiments

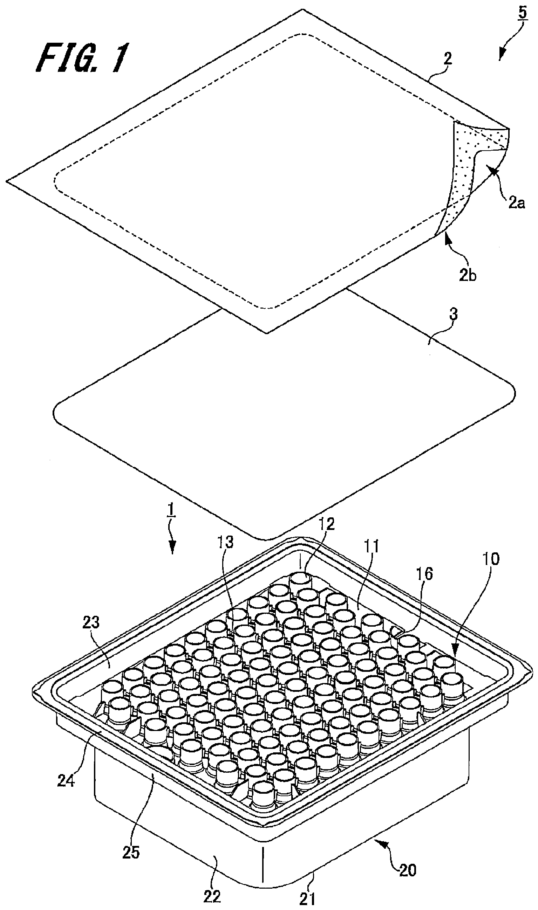

Embodiments of a syringe holding member, a syringe package body and a method for assembling a syringe package body according to the present invention will be described with reference to Figs. 1 to 29. In the drawings, common members are denoted by the same reference numerals. Moreover, the present invention is not limited to the following embodiments.

### 1. First Exemplary Embodiment

### 1-1. Configuration of syringe package body

First, a schematic configuration of a syringe package body according to a first exemplary embodiment (hereinafter, "the present example") will be described with reference to Fig. 1.

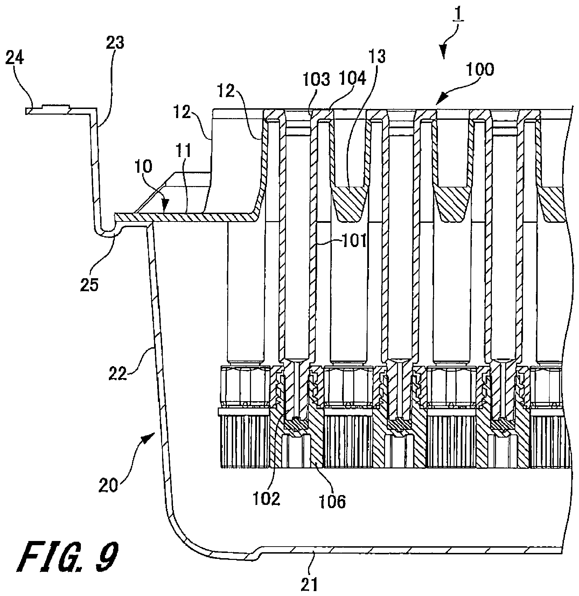

Fig. 1 is a perspective view of a syringe package body of the present example. Note that Fig. 1 omits illustration of the syringe 100 (see Fig. 9).

The syringe package body 5 of the present example includes a syringe storage container 1 and the syringe 100 (see Fig. 9) (not illustrated) stored in the syringe storage container 1. The syringe package body 5 includes a sealing member 2 and an inner sheet 3. The syringe storage container 1 is used for simultaneously conveying and storing a plurality of the syringes 100 (see Fig. 9). As illustrated in Fig. 1, the syringe storage container 1 includes: a syringe holding member 10 that holds a syringe; and a container main body 20 that stores the syringe holding member 10 holding the plurality of syringes.

### [Container main body]

The container main body 20 is formed in a substantially quadrangular shape hollow with an open upper surface. The container main body 20 includes: a peripheral wall portion 22 including an upper end portion and a lower end portion; and a bottom portion 21 continuous with the lower end portion of the peripheral wall portion 22 and surrounded by the lower end portion. The container main body 20 further includes; an opening portion 23 surrounded by the upper end portion of the peripheral wall portion 22; and a flange 24 extending substantially perpendicularly from the upper end portion of the peripheral wall 22 and surrounding the peripheral wall portion 22.

Furthermore, a mounting shelf 25 on which the syringe holding member 10 is mounted is formed on the peripheral wall portion 22. The mounting shelf 25 is formed in a middle portion between the upper end portion and the lower end portion of the peripheral wall portion 22. The mounting shelf 25 is bent substantially perpendicularly in a direction away from the lower end portion of the peripheral wall portion 22. The upper end portion of the peripheral wall portion 22 is continuous substantially perpendicularly from the outer end portion of the mounting shelf 25.

The flange 24 is substantially orthogonal to the upper end of the peripheral wall portion 22, and is formed in a quadrangular frame shape. At shipment of the syringe storage container 1, the sealing member 2 is attached to the flange 24. With this operation, an internal space of the container main body 20 is sealed by the sealing member 2.

### [Syringe holding member]

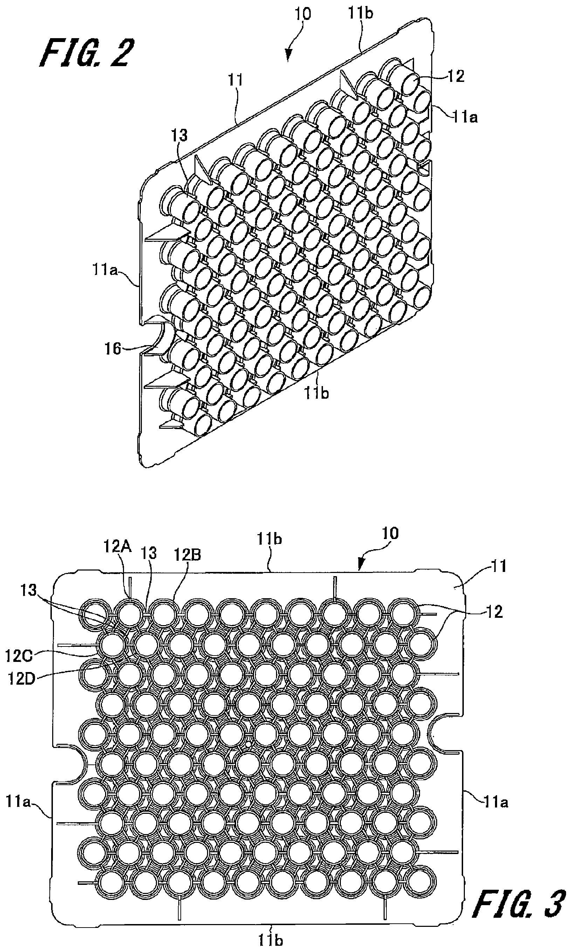

Next, the syringe holding member 10 will be described with reference to Figs. 2 to 8.

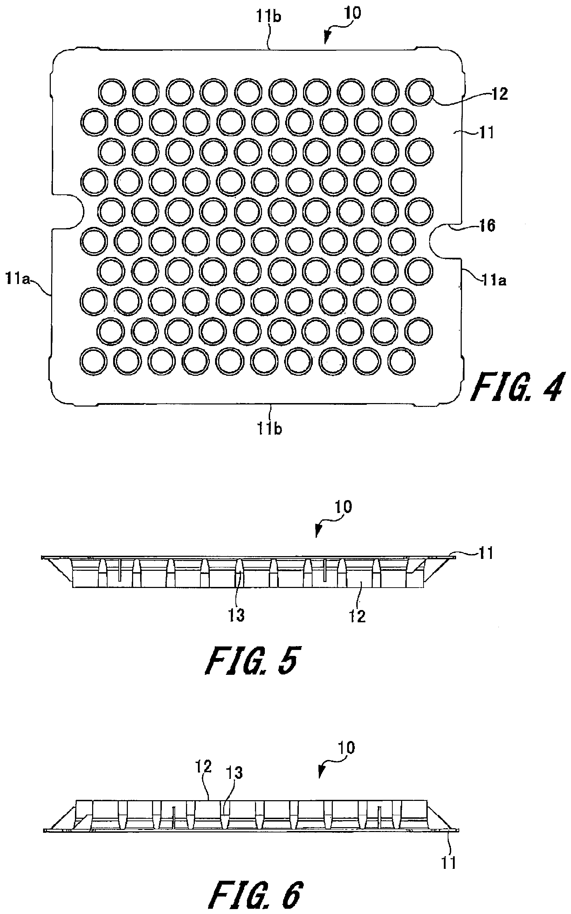

Fig. 2 is a front perspective view illustrating the syringe holding member 10. Fig. 3 is a front view illustrating the syringe holding member 10. Fig. 4 is a rear view illustrating the syringe holding member 10. Fig. 5 is a plan view illustrating the syringe holding member 10.

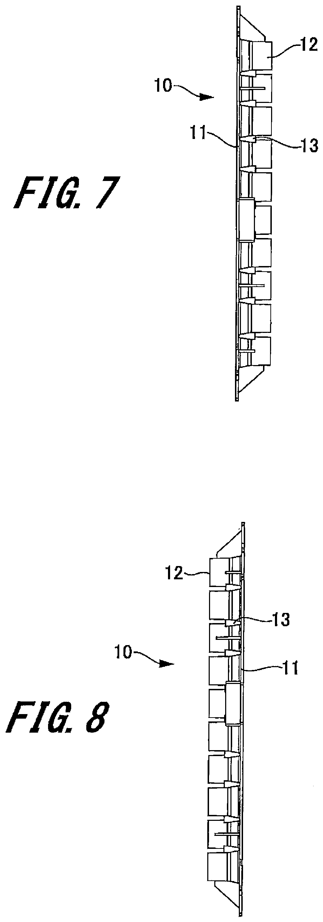

Fig. 6 is a bottom view illustrating the syringe holding member 10. Fig. 7 is a left side view illustrating the syringe holding member 10. Fig. 8 is a right side view illustrating the syringe holding member 10.

As illustrated in Figs. 2 to 8, the syringe holding member 10 includes a substantially flat plate shaped base plate portion 11, a plurality of holding tube portions 12 protruding from the upper surface of the base plate portion 11, and a plurality of reinforcement ribs 13. The base plate portion 11 is formed in a substantially rectangular shape. The base plate portion 11 includes a short side 11a and a long side 11b. The size of the base plate portion 11 has been set to be smaller than the opening portion 23 of the container main body 20 and set to be larger than a region formed by the mounting shelf 25 of the container main body 20. Specifically, the size of the upper surface of the base plate portion 11 has been set to include the long side 11b set to a range of 220 mm to 240 mm and the short side 11a set to a range of 190 mm to 210 mm. In addition, the thickness of the base plate portion 11 has been set to a range of 0.8 mm to 2.5 mm.

A cutout portion 16 is formed on two opposite short sides of the base plate portion 11. The cutout portion 16 is formed by cutting out a portion of the short side 11a of the base plate portion 11 in a substantially semicircular shape. The user can easily take out the syringe holding member 10 mounted on the container main body 20 by inserting a finger into the cutout portion 16.

The holding tube portion 12 is formed in a cylindrical shape. The holding tube portion 12 protrudes upward from the upper surface of the base plate portion 11. Furthermore, the cylindrical hole of the holding tube portion 12 passes through the base plate portion 11. The syringe 100 is inserted into the cylindrical hole of the holding tube portion 12. The opening diameter of the cylindrical hole of the holding tube portion 12 has been set to be larger than the outer diameter of a syringe main body 101 described below in the syringe 100. Specifically, the opening diameter of the holding tube portion 12 has been set to a range of 12.0 mm to 12.5 mm.

The plurality of holding tube portions 12 is regularly arranged at equal intervals on the upper surface of the base plate portion 11. Specifically, the plurality of holding tube portions 12 is arranged in a staggered manner with a predetermined interval in the base plate portion 11. That is, except for the holding tube portion 12 arranged on the outer periphery, the plurality of holding tube portions 12 is arranged such that the six holding tube portions 12 are arranged at substantially equal intervals around one holding tube portion 12, specifically at intervals of about 60 degrees. In addition, the plurality of holding tube portions 12 is arranged in a plurality of rows in a longitudinal direction (first direction) of the base plate portion 11, with the plurality of holding tube portions 12 linearly arranged with a predetermined interval in each of the plurality of rows. In addition, the plurality of holding tube portions 12 is arranged in a plurality of rows in a lateral direction (second direction) of the base plate portion 11 with the plurality of holding tube portions 12 arranged in a continuous zigzag pattern with a predetermined interval in each of the plurality of rows. This arrangement enables substantially equal distances between the adjacent holding tube portions 12 and equal distances between the syringes 100 inserted and held in the holding tube portion 12. This makes it possible to prevent the adjacent syringe 100 from coming in contact with each other during conveyance of the syringe storage container 1.

Here, the continuous zigzag pattern means a shape including any three consecutive holding tube portions 12 being arranged with "alternate turns" in a plurality of holding tube portions 12 arranged in a row. The plurality of holding tube portions 12 thus arranged forms a row in the lateral direction (second direction) of the base plate portion 11. In addition, the rows of a plurality of holding tube portions 12 arranged in a zigzag pattern continuously in the lateral direction of the base plate portion 11 include two types of rows in which the directions of points of "alternate turns" are opposite to each other. Furthermore, the rows of the plurality of holding tube portions 12 positioned in the vicinity of both ends in the longitudinal direction of the base plate portion 11 constitute one of these two types of rows alone.

The reinforcement rib 13 protrudes substantially perpendicularly from the upper surface of the base plate portion 11. The reinforcement rib 13 is formed in a plate shape. As illustrated in Fig. 3, the plurality of reinforcement ribs 13 is arranged so as to connect each of the plurality of holding tube portions 12 and each of the other adjacent holding tube portions 12. In addition, a plurality of the two reinforcement ribs 13 is provided between two adjacent predetermined holding tube portions 12 among the plurality of holding tube portions 12. This makes it possible to increase the strength of the base plate portion 11 as compared with the case where each of the plurality of holding tube portions 12 is connected with each of the other adjacent holding tube portions 12 by one reinforcement rib 13.

The holding tube portions 12 arranged on the outer periphery of the plurality of holding tube portions 12 are connected by one reinforcement rib 13. In addition, holding tube portions 12A and 12B adjacent to each other in the longitudinal direction of the base plate portion 11 are connected to each other by one reinforcement rib 13. In contrast, two parallel reinforcement ribs 13 are used to connect between the holding tube portion 12A and the holding tube portion 12C arranged in the adjacent row in the lateral direction of the base plate portion 11 with respect to the row having the holding tube portion 12A arranged in the longitudinal direction of the base plate portion 11. That is, the two holding tube portions 12 namely, the holding tube portion 12A and the holding tube portion 12C, are one example of the predetermined two holding tube portions 12 connected by the two reinforcement ribs 13. Meanwhile, a holding tube portion 12D adjacent to a holding tube portion 12C in the longitudinal direction of the base plate portion 11 and arranged in the row adjacent to the holding tube portion 12A is connected with the holding tube portion 12A by one reinforcement rib 13.

Furthermore, the holding tube portion 12B and the holding tube portion 12D are connected with each other by two parallel reinforcement ribs 13. That is, the two holding tube portions 12 namely, the holding tube portion 12B and the holding tube portion 12D, are also one example of the predetermined two holding tube portions 12 connected by the two reinforcement ribs 13. A regular arrangement of the plurality of the holding tube portions 12A to 12D achieves a regular arrangement of a plurality of predetermined two holding tube portions 12 connected by the two reinforcement ribs 13. This arrangement makes it possible to further increase the strength of the base plate portion 11.

Among the two types of rows of the plurality of holding tube portions 12 arranged in a continuous zigzag pattern in the lateral direction of the base plate portion 11, one type of row includes sets of the two holding tube portions 12 connected by two parallel reinforcement ribs 13, as continuous arrangement of the sets without interruption extending from one end to the other end of the row. That is, about half of the plurality of rows of the plurality of holding tube portions 12 arranged in the lateral direction of the base plate portion 11 are formed with reinforcement rows of two predetermined holding tube portions 12 connected by two reinforcement ribs 13 continuously arranged from one end to the other end in each of rows. This arrangement suppresses deformation of the base plate portion 11 in the lateral direction of the base plate portion 11. Furthermore, a plurality of such reinforcement rows is formed in the longitudinal direction of the base plate portion 11. This arrangement further suppresses deformation of the base plate portion 11 in the lateral direction of the base plate portion 11. Note that simply one such reinforcement row may be formed in the longitudinal direction of the base plate portion 11.

Here, an outer peripheral portion of the base plate portion 11 is less easily deformed than the inner portion including arrangement of a large number of the syringes 100. Therefore, it is possible to ensure the strength of the outer peripheral portion of the base plate portion 11 simply by connecting the holding tube portions 12 arranged on the outer periphery by one reinforcement rib 13. In addition, the predetermined two holding tube portions 12 connected by the two reinforcement ribs 13 may be arranged regularly as described above, or randomly.

It is preferable that the container main body 20 and the syringe holding member 10 are not deformed or are less easily deformed by sterilization treatment involving a heat load using high temperature steam or gas such as high pressure steam sterilization (autoclave) or ethylene oxide gas (EOG) sterilization. Examples of the materials of the container main body 20 and the syringe holding member 10 include a resin having excellent durability such as polypropylene, polystyrene, polyethylene, polycarbonate, ABS resin, and PET. High pressure steam at 121°C or higher is normally used for high pressure steam sterilization (autoclave). Therefore, when sterilization treatment with high-pressure steam is performed, a resin having a normal heat-resistant temperature of 121°C or higher is preferable as the material of the container main body 20 and the syringe holding member 10. Examples of such a material include polypropylene, polycarbonate, and PET among the above resins. In addition, the container main body 20 and the syringe holding member 10 are preferably substantially transparent or semi-transparent in order to ensure the visibility of the interior of the container main body 20. Examples of these are polypropylene, polycarbonate, polystyrene, and PET among the above-described resins.

### [Sealing member]

As illustrated in Fig. 1, the sealing member 2 is formed into a quadrangular sheet shape substantially equal to an outline of an outer periphery of the flange 24 in the container main body 20. The sealing member 2 is thermally welded to the flange 24 of the container main body 20 using an adhesive. A surface of the sealing member 2 opposite to the container main body 20 includes an adhesive non-application region 2a and an adhesive application region 2b. A thermoplastic adhesive (hot melt) is applied to the adhesive application region 2b. The adhesive application region 2b is brought into contact with the flange 24 of the container main body 20. Thereafter, a contact portion between the sealing member 2 and the flange 24 is heated by the mold, whereby the adhesive is melted and the sealing member 2 and the flange 24 are thermally welded (bonded) to each other.

The adhesive non-application region 2a is surrounded by the adhesive application region 2b. No adhesive is applied to the adhesive non-application region 2a. This adhesive non-application region 2a is set in a region opposite to a filling port 103 (described below) of the syringe 100 held by the syringe holding member 10.

Examples of the sealing member 2 include a moisture permeable waterproof sheet capable of sterilization treatment using high temperature steam or gas such as high pressure steam sterilization (autoclave) and ethylene oxide gas (EOG). That is, the sealing member 2 is formed of a material including gas permeability and bacteria impermeability, that is, permeable to gas for sterilization and impermeable to fine particles such as bacteria and viruses.

### [Inner sheet]

The inner sheet 3 is formed in a quadrangular sheet shape. The area of the inner sheet 3 has been set to a size that covers the filling port 103 side (described below) of the plurality of syringes 100 held by the syringe holding member 10. The inner sheet 3 is mounted above a flange portion 104 of the syringe 100 held by the syringe holding member 10.

The inner sheet 3 is formed of a material including gas permeability and bacteria impermeability, through which gas for sterilization is permeable and fine particles such as bacteria and viruses cannot pass. Furthermore, the inner sheet 3 may be constituted with a metallized sheet or a metal sheet that shields electron beams, ultraviolet rays, or the like. This makes it possible to prevent the syringe 100 from being irradiated with electron beams or ultraviolet rays in sterilizing the outer peripheral surface of the syringe package body 5 with electron beams or ultraviolet rays before filling the medicinal solution.

### [Syringe]

Next, the syringe 100 will be described with reference to Fig. 9.

Fig. 9 is a cross sectional view illustrating a state in which a syringe is stored in a syringe storage container.

As illustrated in Fig. 9, the syringe 100 includes a syringe main body 101 formed of a hollow cylindrical body and a discharge portion 102 formed on the distal side of the syringe main body 101. The filling port 103 for filling a medicinal solution in a medicine chamber of the syringe main body 101 is formed on the proximal side of the syringe main body 101 opposite to the discharge portion 102.

The flange portion 104 is formed around the filling port 103 of the syringe main body 101. At least a portion of the outer diameter of the flange portion 104 is formed to be larger than the diameter of the cylindrical hole of the holding tube portion 12 of the syringe holding member 10. Therefore, when the syringe 100 is inserted into the cylindrical hole of the holding tube portion 12, the flange portion 104 of the syringe 100 is mounted on an upper end portion of the holding tube portion 12.

A cap member 106 is attached to the discharge portion 102. The cap member 106 is formed in a substantially cylindrical shape. The outer diameter of the cap member 106 is formed to be smaller than the diameter of the cylindrical hole of the holding tube portion 12. The cap member 106 is detachably attached to the discharge portion 102 and closes the discharge port of the discharge portion 102.

### 1-2. Method for assembling syringe package body

Next, a method for assembling the syringe package body 5 including the above-described configuration will be described.

First, as illustrated in Fig. 9, the syringe 100 is inserted into the cylindrical hole of the holding tube portion 12 from the discharge portion 102 side on which the cap member 106 is mounted. The flange portion 104 is mounted on the upper end portion of the holding tube portion 12, allowing the syringe 100 to be held by the syringe holding member 10.

Next, as illustrated in Fig. 1, the base plate portion 11 of the syringe holding member 10 is mounted on the mounting shelf 25 of the container main body 20. This allows the syringe holding member 10 to be supported by the container main body 20. Note that it is allowable to store the syringe holding member 10 in the storage space of the container main body 20, and then, after assembling the syringe storage container 1, the syringe 100 may be inserted into the cylindrical hole of the holding tube portion 12 of the syringe holding member 10.

Next, the inner sheet 3 is mounted on the flange portions 104 of the plurality of syringes 100 held by the holding tube portion 12 of the syringe holding member 10. This allows the filling port 103 (flange portion 104) side of the plurality of syringes 100 held by the syringe holding member 10 to be covered with the inner sheet 3.

Next, the sealing member 2 is mounted on the flange 24 of the container main body 20. Then, a receiving jig (not illustrated) is brought into contact with a lower surface of the flange 24, and the sealing member 2 is pressed by a warmed metal mold (not illustrated). This operation melts the adhesive applied to the sealing member 2 to thermally weld (bond) the sealing member 2 to the flange 24, so as to seal the opening portion 23 of the container main body 20 by the sealing member 2.

Next, sterilization treatment involving a heat load using high temperature steam or gas such as high pressure steam sterilization (autoclave) or ethylene oxide gas (EOG) sterilization is applied onto the syringe storage container 1, the sealing member 2, the inner sheet 3 and the plurality of syringes 100. Here, a plurality of reinforcement ribs 13 is formed on the base plate portion 11 of the syringe holding member 10. Each of the plurality of reinforcement ribs 13 connects each of the plurality of adjacent holding tube portions 12 with each other. Two reinforcement ribs 13 are provided between two predetermined holding tube portions 12 adjacent to each other. This arrangement makes it possible to increase the strength of the base plate portion 11 and to prevent deformation of the base plate portion 11 due to the load of the syringe 100 and heat during sterilization treatment.

In addition, there is provided reinforcement rows of two predetermined holding tube portions 12 continued with two reinforcement ribs 13 continuously arranged from the vicinity of one end to the vicinity of the other end in the lateral direction of the base plate portion 11. A plurality of the reinforcement rows is provided in the longitudinal direction of the base plate portion 11. This configuration suppresses deformation of the base plate portion 11 in the lateral direction of the base plate portion 11, making is possible to prevent deformation of the base plate portion 11 in the lateral direction of the base plate portion 11 due to the load of the syringe 100 and the heat during the sterilization treatment.

As described above, the base plate portion 11 is easily deformed by heat in a case where the base plate portion 11 has dimensions of a long side 11b set to a range of 220 mm to 240 mm and a short side 11a set to a range of 190 mm to 210 mm on an upper surface and a thickness of the base plate portion 11 set to a range of 0.8 mm to 2.5 mm. This makes it important to connect between the two predetermined holding tube portions 12 adjacent to each other by the two reinforcement ribs 13 to increase the strength of the base plate portion 11.

An assembly step of the syringe package body 5 is completed together with completion of the sterilization treatment. The syringe package body 5 that has been completed its assembling work is conveyed to a work place for filling the medicinal solution. Before filling the medicinal solution, the outer surface of the syringe package body 5 is sterilized with electron beams or ultraviolet rays. At this time, the inner sheet 3 is interposed between the syringe 100 and the sealing member 2. This enables the inner sheet 3 to prevent excessive irradiation of the syringe 100 with electron beams or ultraviolet rays.

The sealing member 2 and the inner sheet 3 are removed when the medicine chamber of the syringe main body 101 is filled with a medicinal solution. Then, the medicine chamber of the syringe main body 101 is filled with the medicinal solution with the syringe 100 held by the syringe holding member 10. As described above, the syringe holding member 10 that holds the syringe 100 is less easily deformed with increased strength. This prevents deformation of the syringe holding member 10 during the sterilization treatment involving a heat load and prevents a change in the orientation of the filling port 103 of the syringe 100 and intervals of the syringes 100 before and after sterilization. Therefore, it is possible to reliably fill the medicine chamber of the syringe main body 101 with the medicinal solution at the time of filling operation of the medicinal solution.

After completion of the filling operation of the medicinal solution into the medicine chamber of the syringe main body 101, the gasket is capped into the cylindrical hole of the syringe main body 101 from the filling port 103 in a state where the syringe 100 is held by the syringe holding member 10. Since deformation of the syringe holding member 10 is suppressed even at the capping operation, the gasket can be reliably capped into the cylindrical hole of the syringe main body 101.

### 2. Second Exemplary Embodiment

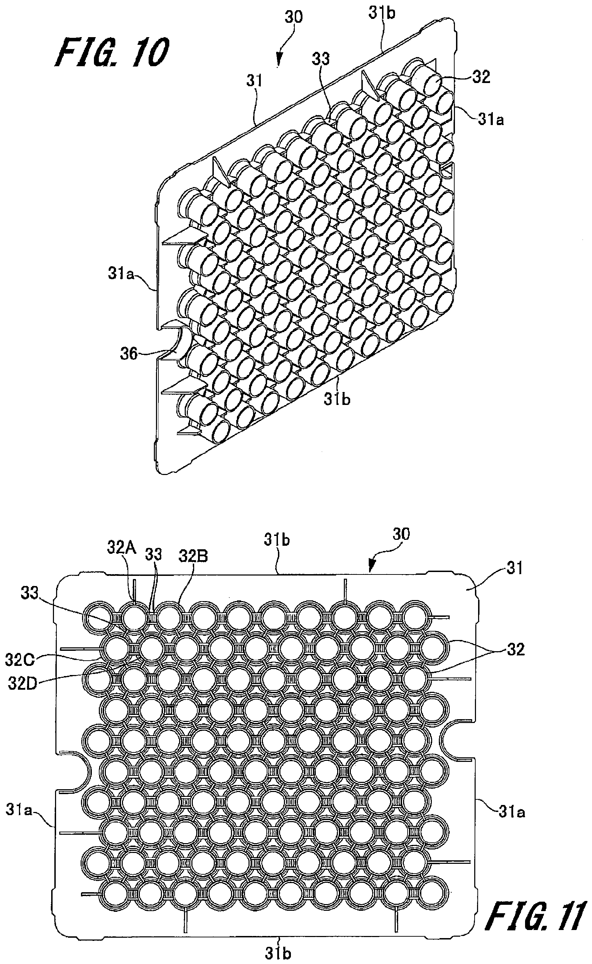

Next, a syringe holding member according to a second exemplary embodiment will be described with reference to Figs. 10 to 16.

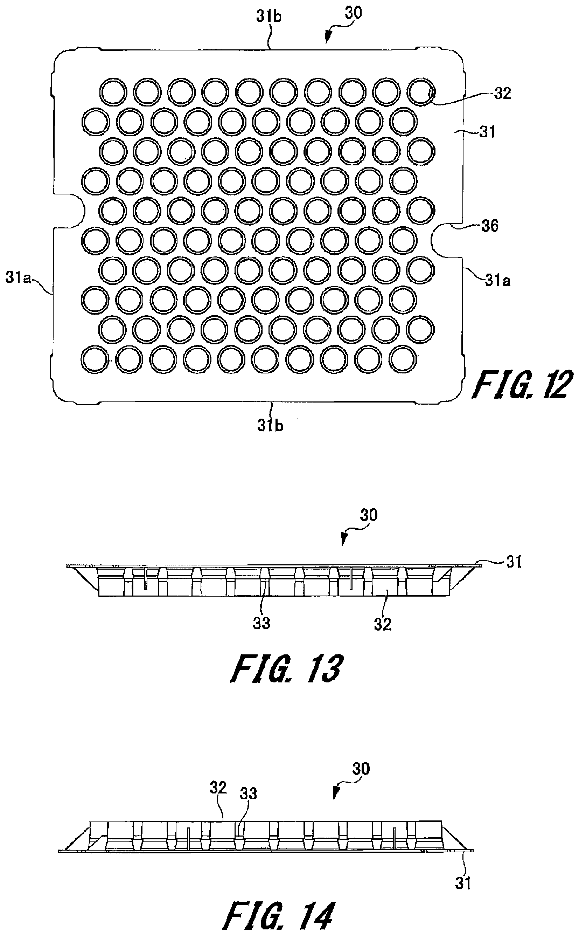

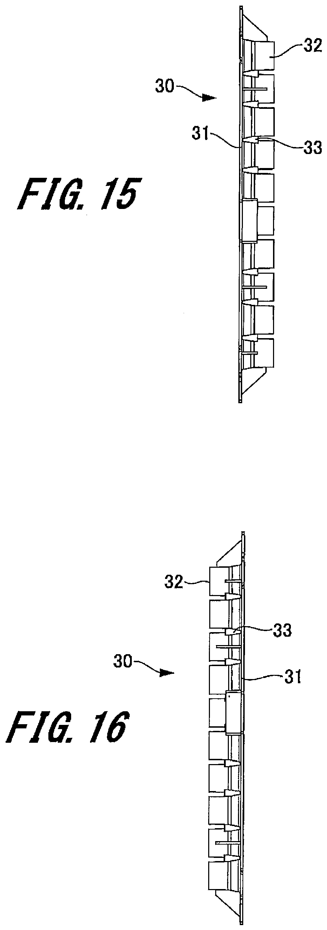

Fig. 10 is a front perspective view illustrating the syringe holding member. Fig. 11 is a front view illustrating the syringe holding member. Fig. 12 is a rear view illustrating the syringe holding member. Fig. 13 is a plan view illustrating the syringe holding member. Fig. 14 is a bottom view illustrating the syringe holding member. Fig. 15 is a left side view illustrating the syringe holding member. Fig. 16 is a right side view illustrating the syringe holding member.

A syringe holding member 30 according to the second embodiment differs from the syringe holding member 10 according to the first exemplary embodiment in the arrangement of the two predetermined holding tube portions connected by two reinforcement ribs. Therefore, duplicate descriptions are omitted for portions common to the syringe holding member 10 according to the first exemplary embodiment.

As illustrated in Figs. 10 to 16, the syringe holding member 30 includes a base plate portion 31, a plurality of holding tube portions 32, and a plurality of reinforcement ribs 33. A cutout portion 36 is formed in two opposite short sides 31a of the base plate portion 31.

As illustrated in Fig. 11, the plurality of holding tube portions 32 is arranged in a staggered manner with a predetermined interval in the base plate portion 31. In addition, the plurality of holding tube portions 32 is arranged in a plurality of rows in a longitudinal direction (first direction) of the base plate portion 31, with the plurality of holding tube portions 32 linearly arranged with a predetermined interval in each of the plurality of rows. In addition, the plurality of holding tube portions 32 is arranged in a plurality of rows in a lateral direction of the base plate portion 31 with the plurality of holding tube portions 32 arranged in a continuous zigzag pattern with a predetermined interval in each of the plurality of rows.

As illustrated in Fig. 11, the plurality of reinforcement ribs 33 is arranged so as to connect each of the plurality of holding tube portions 32 and each of the other adjacent holding tube portions 32. In addition, two reinforcement ribs 33 are provided between two adjacent predetermined holding tube portions 32 among the plurality of holding tube portions 32. This makes it possible to increase the strength of the base plate portion 31 as compared with the case where each of the plurality of holding tube portions 32 is connected with each of the other adjacent holding tube portions 32 by one reinforcement rib 33.

In addition, holding tube portions 32A and 32B adjacent to each other in the longitudinal direction of the base plate portion 31 are connected to each other by two parallel reinforcement ribs 33. That is, the two holding tube portions 32 namely, the holding tube portion 32A and the holding tube portion 32B, are one example of the predetermined two holding tube portions 32 connected by the two reinforcement ribs 33. In contrast, one reinforcement rib 33 is used to connect between the holding tube portion 32A and each of the holding tube portion 32C and the holding tube portion 32D, arranged in the adjacent row in the lateral direction of the base plate portion 31 with respect to the row having the holding tube portion 32A arranged in the longitudinal direction of the base plate portion 31. A regular arrangement of the plurality of the holding tube portions 32A to 32D achieves a regular arrangement of predetermined two holding tube portions 32 connected by the two reinforcement ribs 33. This arrangement makes it possible to further increase the strength of the base plate portion 31.

The row of the plurality of holding tube portions 32 linearly arranged in the longitudinal direction of the base plate portion 31 includes arrangements of sets of the two holding tube portions 32 connected by two parallel reinforcement ribs 33 as continuous arrangements of the sets without interruption extending from one end to the other end of the row. That is, each of the plurality of rows of the plurality of holding tube portions 32 arranged in the longitudinal direction of the base plate portion 31 is formed with reinforcement rows of two predetermined holding tube portions 32 connected by two reinforcement ribs 33 continuously arranged from one end to the other end in each of rows. This arrangement suppresses deformation of the base plate portion 31 in the longitudinal direction of the base plate portion 11. Furthermore, a plurality of such reinforcement rows is formed in the lateral direction of the base plate portion 31. This arrangement further suppresses deformation of the base plate portion 31 in the longitudinal direction of the base plate portion 31. Note that simply one such reinforcement row may be formed in the lateral direction of the base plate portion 31.

Furthermore, among the plurality of holding tube portions 32 arranged on the outer periphery, the two holding tube portions 32 adjacent to each other in the longitudinal direction are connected by two reinforcement ribs 33, while two holding tube portions 32 adjacent to each other in the lateral direction are connected by one reinforcement rib 33. Both end portions of the outer peripheral portion of the base plate portion 31 in the longitudinal direction are less easily deformed in particular among portions of the base plate portion 31. Therefore, it is possible to ensure the strength of the both end portions of the base plate portion 31 in the longitudinal direction simply by connecting the holding tube portions 32 arranged on the both end portions with one reinforcement rib 33.

Since other configurations are similar to the configuration of the syringe holding member 10 according to the first exemplary embodiment, description will be omitted. The above-configured syringe holding member 30 can also obtain the similar operational effects as syringe holding member 10 according to the first exemplary embodiment described above.

### 3. Third Exemplary Embodiment

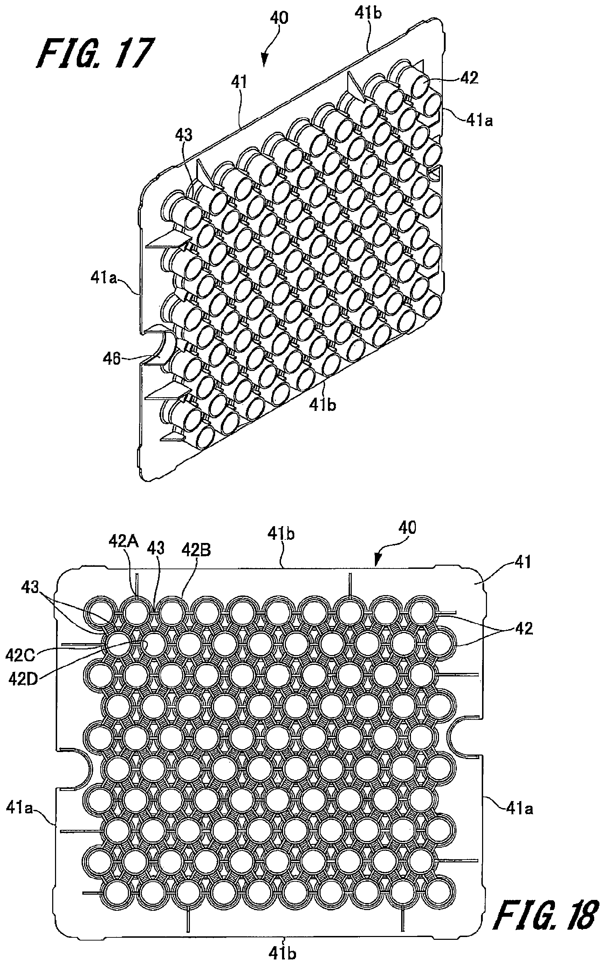

Next, a syringe holding member according to a third exemplary embodiment will be described with reference to Figs. 17 to 23.

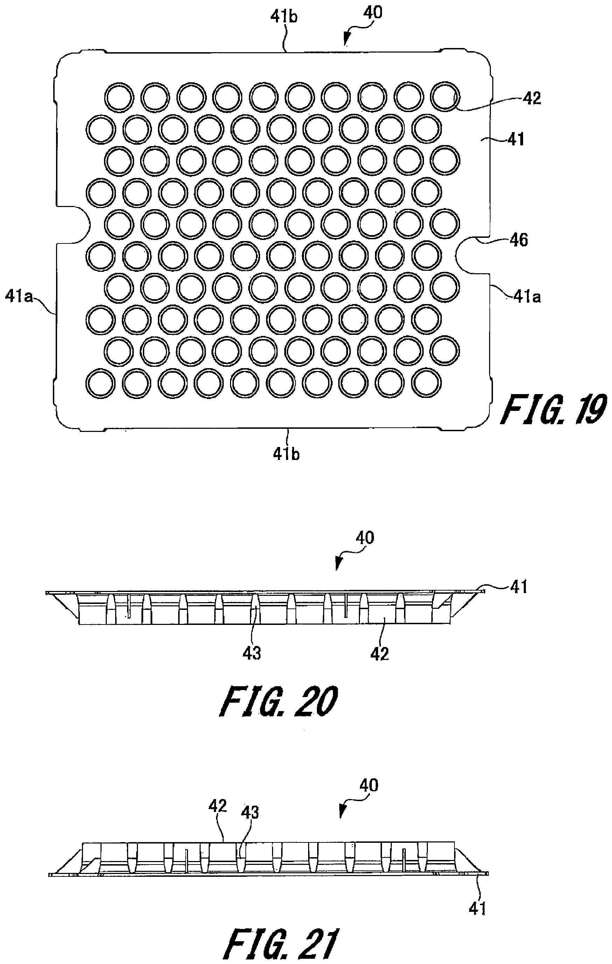

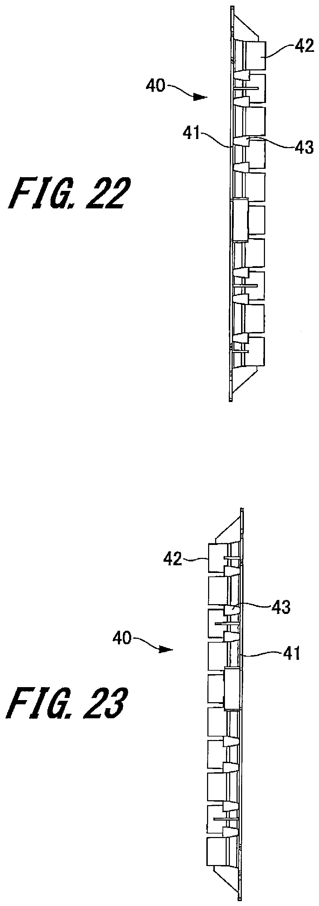

Fig. 17 is a front perspective view illustrating the syringe holding member. Fig. 18 is a front view illustrating the syringe holding member. Fig. 19 is a rear view illustrating the syringe holding member. Fig. 20 is a plan view illustrating the syringe holding member. Fig. 21 is a bottom view illustrating the syringe holding member. Fig. 22 is a left side view illustrating the syringe holding member. Fig. 23 is a right side view illustrating the syringe holding member.

A syringe holding member 40 according to the third embodiment differs from the syringe holding member 10 according to the first exemplary embodiment in the arrangement of the two predetermined holding tube portions connected by two reinforcement ribs. Therefore, duplicate descriptions are omitted for portions common to the syringe holding member 10 according to the first exemplary embodiment.

As illustrated in Figs. 17 to 23, the syringe holding member 40 includes a base plate portion 41, a plurality of holding tube portions 42, and a plurality of reinforcement ribs 43. A cutout portion 46 is formed in two opposite short sides 41a of the base plate portion 41.

As illustrated in Fig. 18, the plurality of holding tube portions 42 is arranged in a staggered manner with a predetermined interval in the base plate portion 41. In addition, the plurality of holding tube portions 42 is arranged in a plurality of rows in a longitudinal direction (first direction) of the base plate portion 41, with the plurality of holding tube portions 42 linearly arranged with a predetermined interval in each of the plurality of rows. In addition, the plurality of holding tube portions 42 is arranged in a plurality of rows in a lateral direction of the base plate portion 41 with the plurality of holding tube portions 42 arranged in a continuous zigzag pattern with a predetermined interval in each of the plurality of rows.

As illustrated in Fig. 18, the plurality of reinforcement ribs 43 is arranged so as to connect each of the plurality of holding tube portions 42 and each of the other adjacent holding tube portions 42. In addition, two reinforcement ribs 43 are provided between two adjacent predetermined holding tube portions 42 among the plurality of holding tube portions 42. This makes it possible to increase the strength of the base plate portion 41 as compared with the case where each of the plurality of holding tube portions 42 is connected with each of the other adjacent holding tube portions 42 by one reinforcement rib 43.

In addition, holding tube portions 42A and 42B adjacent to each other in the longitudinal direction of the base plate portion 41 are connected to each other by one reinforcement rib 43. In contrast, two parallel reinforcement ribs 43 are used to connect between the holding tube portion 42A and the holding tube portion 42C arranged in the adjacent row in the lateral direction of the base plate portion 41 with respect to the row having the holding tube portion 42A arranged in the longitudinal direction of the base plate portion 41. That is, the two holding tube portions 42 namely, the holding tube portion 42A and the holding tube portion 42C, are one example of the predetermined two holding tube portions 42 connected by the two reinforcement ribs 43.

Similarly, a holding tube portion 42D adjacent to a holding tube portion 42C in the longitudinal direction of the base plate portion 41 and adjacent to the holding tube portion 42A in the lateral direction is connected with the holding tube portion 42A by two parallel reinforcement ribs 43. That is, the two holding tube portions 42 namely, the holding tube portion 42A and the holding tube portion 42D, are also one example of the predetermined two holding tube portions 42 connected by the two reinforcement ribs 43.

Moreover, a holding tube portion 42B adjacent to the holding tube portion 42A in the longitudinal direction of the base plate portion 41 and adjacent to the holding tube portion 42D in the lateral direction is connected with the holding tube portion 42D by two parallel reinforcement ribs 43. That is, the two holding tube portions 42 namely, the holding tube portion 42B and the holding tube portion 42D, are also one example of the predetermined two holding tube portions 42 connected by the two reinforcement ribs 43.

A regular arrangement of the plurality of the holding tube portions 42A to 42D achieves a regular arrangement of a plurality of predetermined two holding tube portions 42 connected by the two reinforcement ribs 13. This arrangement makes it possible to further increase the strength of the base plate portion 41.

Moreover, both the two types of rows of the plurality of holding tube portions 42 arranged in a continuous zigzag pattern in the lateral direction of the base plate portion 41 include sets of the two holding tube portions 42 connected by two parallel reinforcement ribs 43 as continuous arrangement of the sets without interruption extending from one end to the other end of the row. That is, each of the plurality of rows of the plurality of holding tube portions 42 arranged in the lateral direction of the base plate portion 41 is formed with reinforcement rows of two predetermined holding tube portions 42 connected by two reinforcement ribs 43 continuously arranged from one end to the other end in each of rows. This arrangement suppresses deformation of the base plate portion 41 in the lateral direction of the base plate portion 41. Furthermore, a plurality of such reinforcement rows is formed in the longitudinal direction of the base plate portion 41. This arrangement further suppresses deformation of the base plate portion 41 in the lateral direction of the base plate portion 41.

In addition, all of the two types of rows of the plurality of holding tube portions 42 arranged in a continuous zigzag pattern in the lateral direction of the base plate portion 41 constitute the reinforcement rows. With this configuration, the syringe holding member 40 according to the third exemplary embodiment achieves greater strength of the base plate portion 41 in the lateral direction than in the syringe holding member 10 according to the first exemplary embodiment and the syringe holding member 30 according to the second embodiment.

Furthermore, in the two adjacent rows in the longitudinal direction which are adjacent to each other in the lateral direction, the plurality of holding tube portions 42 is arranged in a zigzag pattern in the longitudinal direction of the base plate portion 41. That is, combination of two rows in the longitudinal direction adjacent to each other in the lateral direction would form a zigzag row aligned in the longitudinal direction as continuous rows of the plurality of holding tube portions 42 arranged in a zigzag pattern in the longitudinal direction with a predetermined interval. In this zigzag row in the longitudinal direction, the two parallel holding reinforcement ribs 43 connects between the holding tube portion 42A and each of the two adjacent holding tube portions 42C and 42D adjacent to the holding tube portion 42A. In this manner, in the zigzag row in the longitudinal direction, one holding tube portion 42 and each of the two adjacent holding tube portions 42 are each connected by the two parallel reinforcement ribs 43.

Accordingly, the row of the plurality of holding tube portions 42 arranged in a zigzag pattern in the longitudinal direction of the base plate portion 41 includes arrangements of sets of the two holding tube portions 42 connected by two parallel reinforcement ribs 43 as continuous arrangements of the sets without interruption extending from one end to the other end of the row. That is, each of a plurality of rows of the plurality of holding tube portions 42 arranged in a zigzag pattern in the longitudinal direction of the base plate portion 41 is formed of a reinforcement row connected by the two reinforcement ribs 43. Therefore, the syringe holding member 40 according to the third exemplary embodiment achieves greater strength with respect to the longitudinal direction of the base plate portion 41 than in the syringe holding member 10 according to the first exemplary embodiment.

Since other configurations are similar to the configuration of the syringe holding member 10 according to the first exemplary embodiment, description will be omitted. The above-configured syringe holding member 40 can also obtain the similar operational effects as syringe holding member 10 according to the first exemplary embodiment described above.

### 4. Fourth Exemplary Embodiment

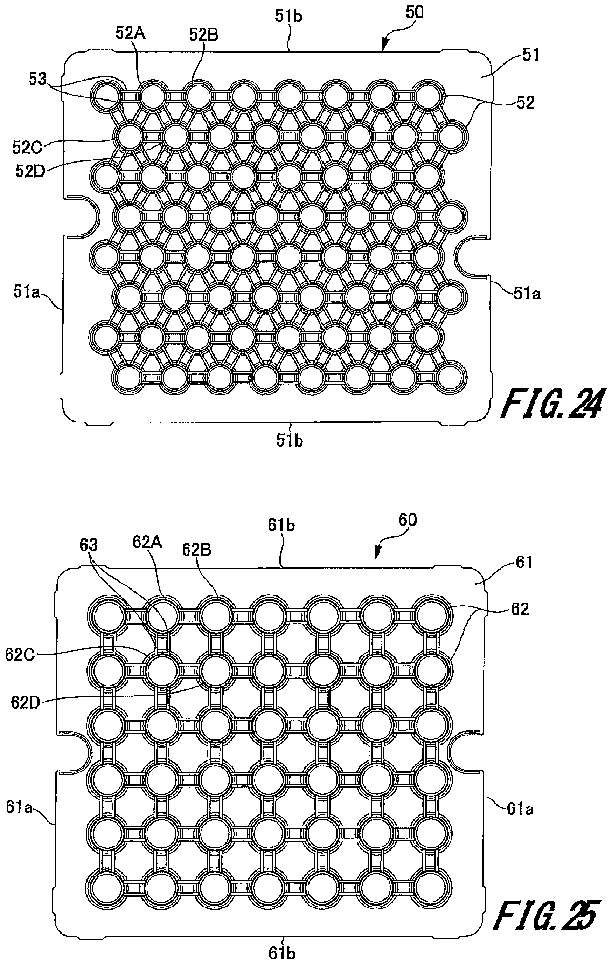

Next, a syringe holding member according to a fourth exemplary embodiment will be described with reference to Fig. 24.

Fig. 24 is a front view illustrating a syringe holding member.

A syringe holding member 50 according to the fourth embodiment differs from the syringe holding member 10 according to the first exemplary embodiment in the number of holding tube portions and the arrangement of the two predetermined holding tube portions connected by two reinforcement ribs. Therefore, duplicate descriptions are omitted for portions common to the syringe holding member 10 according to the first exemplary embodiment.

As illustrated in Fig. 24, the syringe holding member 50 includes a base plate portion 51, a plurality of holding tube portions 52, and a plurality of reinforcement ribs 53. The plurality of holding tube portions 52 is arranged in a staggered manner with a predetermined interval in the base plate portion 51. In addition, the plurality of holding tube portions 52 is arranged in a plurality of rows in a longitudinal direction (first direction) of the base plate portion 51, with the plurality of holding tube portions 52 linearly arranged with a predetermined interval in each of the plurality of rows. In addition, the plurality of holding tube portions 52 is arranged in a plurality of rows in a lateral direction of the base plate portion 51 with the plurality of holding tube portions 52 arranged in a continuous zigzag pattern with a predetermined interval in each of the plurality of rows.

The syringe holding member 50 according to the fourth exemplary embodiment includes 64 holding tube portions 52, fewer than in the syringe holding members 10 according to the first exemplary embodiment. An opening diameter of the cylindrical hole of the holding tube portion 52 has been set to a range of 15.0 mm to 16.5 mm, larger than in the syringe holding member 10 according to the first exemplary embodiment. In the syringe holding member 50 according to the fourth exemplary embodiment, a distance between the adjacent holding tube portions 52 is set slightly longer than in the syringe holding member 10 according to the first exemplary embodiment.

As illustrated in Fig. 24, the plurality of reinforcement ribs 53 is arranged so as to connect each of the plurality of holding tube portions 52 and each of the other adjacent holding tube portions 52. In addition, a plurality of the two reinforcement ribs 53 is provided between all of the adjacent holding tube portions 52. That is, each of the plurality of rows of the plurality of holding tube portions 52 arranged in the longitudinal and lateral directions of the base plate portion 51 is formed with reinforcement rows of two predetermined holding tube portions 52 connected by two reinforcement ribs 53 continuously arranged from one end to the other end in each of rows. This arrangement suppresses deformation of the base plate portion 51 in the longitudinal and lateral directions of the base plate portion 51. Furthermore, a plurality of such reinforcement rows is formed in the longitudinal and lateral directions of the base plate portion 51. This arrangement further suppresses deformation of the base plate portion 51 in the longitudinal and lateral directions of the base plate portion 51.

Furthermore, the syringe holding member 50 according to the fourth exemplary embodiment achieves greater strength than in the syringe holding member 10 according to the first exemplary embodiment and in the syringe holding member 30 according to the second exemplary embodiment and in the syringe holding member 40 according to the third exemplary embodiment.

Since other configurations are similar to the configuration of the syringe holding member 10 according to the first exemplary embodiment, description will be omitted. The above-configured syringe holding member 50 can also obtain the similar operational effects as syringe holding member 10 according to the first exemplary embodiment described above.

### 5. Fifth Exemplary Embodiment

Next, a syringe holding member according to a fifth exemplary embodiment will be described with reference to Fig. 25.

Fig. 25 is a front view illustrating the syringe holding member.

A syringe holding member 60 according to the fifth embodiment differs from the syringe holding member 10 according to the first exemplary embodiment in the number and arrangement of the holding tube portions and the arrangement of the two predetermined holding tube portions connected by two reinforcement ribs. Therefore, duplicate descriptions are omitted for portions common to the syringe holding member 10 according to the first exemplary embodiment.

As illustrated in Fig. 25, the syringe holding member 60 includes a base plate portion 61, a plurality of holding tube portions 62, and a plurality of reinforcement ribs 63. The plurality of holding tube portions 62 is arranged in a matrix with a predetermined interval in the base plate portion 61. That is, the plurality of holding tube portions 62 is arranged linearly in the longitudinal and lateral directions of the base plate portion 61.

In addition, the syringe holding member 60 according to the fifth exemplary embodiment includes 42 holding tube portions 62, fewer than in the syringe holding members 10 according to the first exemplary embodiment. An opening diameter of the cylindrical hole of the holding tube portion 62 has been set to a range of 17.5 mm to 19.5 mm, larger than that of the syringe holding member 10 according to the first exemplary embodiment.

As illustrated in Fig. 25, the plurality of reinforcement ribs 63 is arranged so as to connect each of the plurality of holding tube portions 62 and each of the other adjacent holding tube portions 62. In addition, a plurality of the two reinforcement ribs 63 is provided between all of the adjacent holding tube portions 62. That is, each of the plurality of rows of the plurality of holding tube portions 62 arranged in the longitudinal and lateral directions of the base plate portion 61 is formed with reinforcement rows of two predetermined holding tube portions 62 connected by two reinforcement ribs 63 continuously arranged from one end to the other end in each of rows. This arrangement suppresses deformation of the base plate portion 61 in the longitudinal and lateral directions of the base plate portion 61. Furthermore, a plurality of such reinforcement rows is formed in the longitudinal and lateral directions of the base plate portion 61. This arrangement further suppresses deformation of the base plate portion 61 in the longitudinal and lateral directions of the base plate portion 61.

Since other configurations are similar to the configuration of the syringe holding member 10 according to the first exemplary embodiment, description will be omitted. The above-configured syringe holding member 60 can also obtain the similar operational effects as syringe holding member 10 according to the first exemplary embodiment described above.

Furthermore, in the syringe holding member 60 according to the fifth exemplary embodiment, it is allowable to connect the holding tube portion 62A and the holding tube portion 62D adjacent to the holding tube portion 62A in a diagonal direction inclined with respect to both the longitudinal direction and the lateral direction of the base plate portion 61, by the reinforcement ribs 63.

### 6. Sixth Exemplary Embodiment

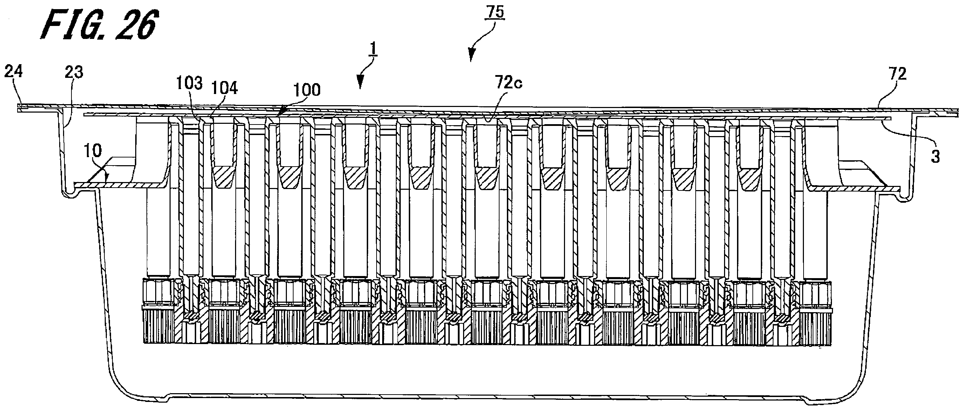

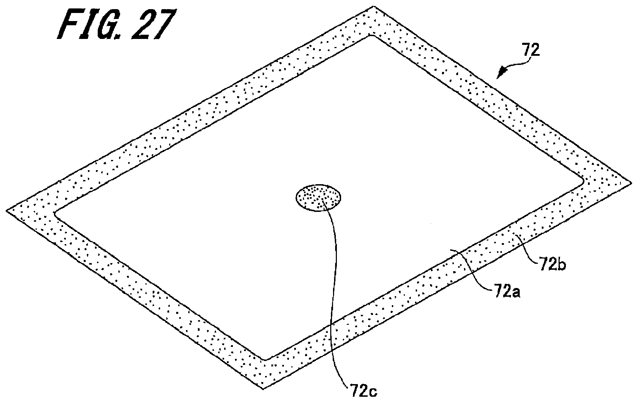

Next, a syringe package body according to a sixth exemplary embodiment will be described with reference to Figs. 26 and 27.

Fig. 26 is a cross sectional view illustrating a syringe package body. Fig. 27 is a plan view illustrating a sealing member.

A syringe package body 75 according to the sixth exemplary embodiment differs from the syringe package body 5 according to the first exemplary embodiment in the configuration of the sealing member. Therefore, the sealing member will be described herein, and duplicate description will be omitted for portions common to the syringe package body 5 according to the first exemplary embodiment.

As illustrated in Figs. 26 and 27, a sealing member 72 is formed in a sheet shape. The sealing member 72 includes, on a surface opposite to the container main body 20: an adhesive non-application region 72a which is a non-joint region; a first adhesive application region 72b for forming a first joint region; and a second adhesive application region 72c for forming a second joint region. The first adhesive application region 72b is formed in a peripheral edge portion similarly to the adhesive application region 2b of the sealing member 2 according to the first exemplary embodiment. A thermoplastic adhesive (hot melt) is applied to the first adhesive application region 72b, so as to be thermally welded with the flange 24 of the container main body 20. That is, the first joint region where the container main body 20 and the sealing member 72 are joined is formed by the first adhesive application region 72b in order to seal the opening portion 23 of the container main body 20 by the sealing member 72.

The adhesive non-application region 72a and the second adhesive application region 72c are provided in an opposite region opposing to the sealing member 72 that is opposed to the inner sheet 3. Note that the first adhesive application region 72b is formed closer to the peripheral edge side of the sealing member 72 than the opposite region.

The second adhesive application region 72c is formed at a central portion of the sealing member 72. A thermoplastic adhesive (hot melt) similar to the adhesive applied to the first adhesive application region 72b is applied to the second adhesive application region 72c. The first adhesive application region 72b and the second adhesive application region 72c have been set to a range of 1% to 10% of the area of opening of the opening portion 23 of the container main body 20.

In assembling the syringe package body 75, the sealing member 72 is mounted on the flange 24 of the container main body 20. At this time, an inner sheet 3 including an outer edge smaller than an inner edge of the opening portion 23 of the container main body 20 is interposed between the sealing member 72 and the syringe 100 stored in the syringe storage container 1. Then, a receiving jig (not illustrated) is brought into contact with the lower surface of the flange 24, and the sealing member 72 is pressed by a warmed metal mold (not illustrated). This operation melts the thermoplastic adhesive applied to the first adhesive application region 72b of the sealing member 72 to thermally weld (bond) the sealing member 72 to the flange 24, so as to seal the opening portion 23 of the container main body 20 by the sealing member 72.

Next, sterilization treatment involving a heat load using high temperature steam or gas such as high pressure steam sterilization (autoclave) or ethylene oxide gas (EOG) sterilization is applied onto the syringe storage container 1, the sealing member 72, the inner sheet 3 and the plurality of syringes 100. The sealing member 72 is pressed by high temperature steam or gas, and its central portion is deformed so as to be recessed to an inner side of the container main body 20. With this configuration, as illustrated in Fig. 26, the inner sheet 3 is bonded to the sealing member 72 by the thermoplastic adhesive applied to the second adhesive application region 72c of the sealing member 72. That is, the second joint region joining the inner sheet 3 with the sealing member 72 is formed by the second adhesive application region 72c bonding the inner sheet 3 with the sealing member 72.

In addition, the sealing member 72 includes an adhesive non-application region 72a to which no adhesive is applied. This transmits high temperature steam or gas through the adhesive non-application region 72a of the sealing member 72, leading to reliable execution of the sterilization treatment on the syringe 100 stored in the syringe storage container 1. That is, the adhesive non-application region 72a is a joint region where the sealing member 72 and the inner sheet 3 are not joined.

An assembly step of the syringe package body 75 is completed together with completion of the sterilization treatment. The syringe package body 75 that has been completed its assembling work is conveyed to a work place for filling the medicinal solution. Before filling the medicinal solution, the outer peripheral surface of the syringe package body 75 is sterilized with electron beams or ultraviolet rays. At this time, the inner sheet 3 is interposed between the syringe 100 and the sealing member 72. This enables the inner sheet 3 to prevent irradiation of the syringe 100 with electron beams or ultraviolet rays.

Then, when the syringe 100 is filled with the medicinal solution, the sealing member 72 is removed from the container main body 20. At this time, the inner sheet 3 is bonded to the sealing member 72 by the adhesive applied to the second adhesive application region 72c. Therefore, it is possible to remove the inner sheet 3 simultaneously with removal of the sealing member 72. This makes it possible to prevent the inner sheet 3 from being left in the container main body 20 when the medicinal solution is filled, leading to higher working efficiency.

Since other configurations are similar to the configuration of the syringe package body 5 according to the first exemplary embodiment, description will be omitted. The above-configured syringe package body 75 including this sealing member 72 can also obtain the similar operational effects as the syringe package body 5 according to the first exemplary embodiment described above.

Furthermore, since an outer edge of the inner sheet 3 is smaller than an inner edge of the opening portion 23 of the container main body 20, a gap is formed between the inner edge of the opening portion 23 of the container main body 20 and the outer edge of the inner sheet 3 (see Fig. 26). In addition, since the opposite region opposing to the sealing member 72 that is opposed to the inner sheet 3 includes a non-joint region where the sealing member 72 and the inner sheet 3 are not joined with each other, a gap is generated also between the sealing member 72 and the inner sheet 3 (see Fig. 26). With formation of this gap, the steam or gas that has passed through the sealing member 72 at the time of sterilization easily enters below the inner sheet 3 in the container main body 20. This enables sterilization treatment using steam or gas to be efficiently performed onto the syringe 100 stored in the syringe storage container 1.

### 7. Seventh Exemplary Embodiment

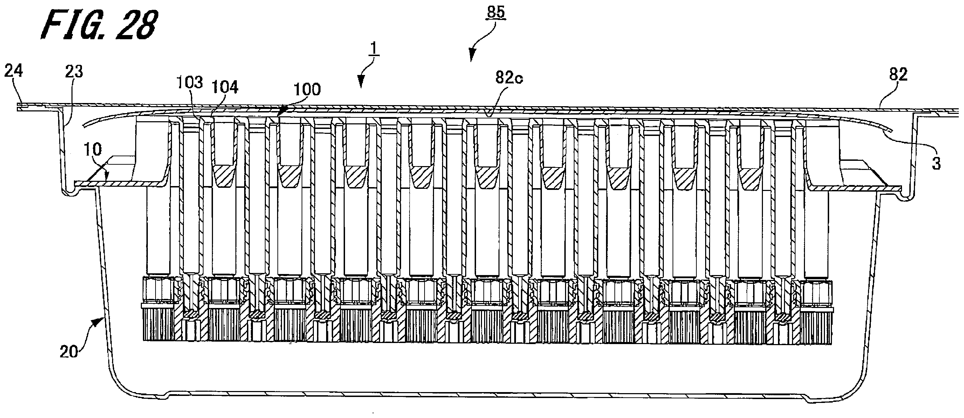

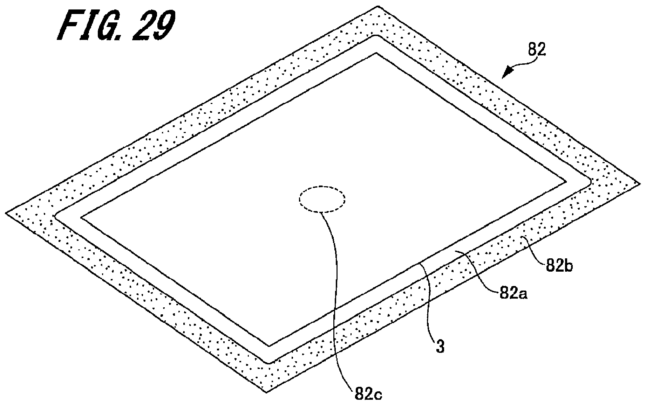

Next, a syringe package body according to a seventh exemplary embodiment will be described with reference to Figs. 28 and 29.

Fig. 28 is a cross sectional view illustrating a syringe package body. Fig. 29 is a plan view illustrating a sealing member.

A syringe package body 85 according to the seventh exemplary embodiment is formed by integrally bonding a sealing member and an inner sheet beforehand. Accordingly, the sealing member and the inner sheet will be described herein, and duplicate descriptions will be omitted for portions common to the syringe package body 5 according to the first exemplary embodiment.

As illustrated in Figs. 28 and 29, the sealing member 82 is formed in a sheet shape. The sealing member 82 includes, on a surface opposite to the container main body 20: an adhesive non-application region 82a representing a non-joint region; a first adhesive application region 82b representing a first joint region; and a second adhesive application region 82c representing a second joint region. The first adhesive application region 82b is formed in a peripheral edge portion similarly to the adhesive application region 2b of the sealing member 2 according to the first exemplary embodiment. A thermoplastic adhesive (hot melt) is applied to the first adhesive application region 82b, so as to be thermally welded with the flange 24 of the container main body 20.

The second adhesive application region 82c is formed in a central portion of the sealing member 82 similarly to the second adhesive application region 72c of the sealing member 72 according to the seventh exemplary embodiment. Adhesive is applied to the second adhesive application region 82c, with the inner sheet 3 being bonded beforehand.

The second adhesive application region 82c to which the inner sheet 3 is bonded is not limited to the central portion of the sealing member 82. For example, a second adhesive application region 82c may be provided at four corners of the adhesive non-application region 82a of the sealing member 82.

Note that the second joint region joining the inner sheet 3 with the sealing member 82 is not limited to the region formed by the second adhesive application region 82c to which the adhesive has been applied. The second joint region may be formed by directly joining the inner sheet 3 with the sealing member 82 by welding such as thermal welding or ultrasonic welding.

At assembly of the syringe package body 85, the sealing member 82 is mounted on the flange 24 of the container main body 20. At this time, as described above, the inner sheet 3 is bonded to the sealing member 82 beforehand. Therefore, the inner sheet 3 is disposed to be opposite to the flange portion 104 of the syringe 100 stored in the syringe storage container 1. This allows the filling port 103 (flange portion 104) side of the plurality of syringes 100 held by the syringe holding member 10 to be covered with the inner sheet 3.

A receiving jig (not illustrated) is brought into contact with the lower surface of the flange 24, and the sealing member 82 is pressed by a warmed metal mold (not illustrated). This operation melts the thermoplastic adhesive applied to the first adhesive application region 82b of the sealing member 82 to thermally weld (bond) the sealing member 82 to the flange 24, so as to seal the opening portion 23 of the container main body 20 by the sealing member 82.

Then, a sterilization treatment involving a heat load using high temperature steam or gas such as high pressure steam sterilization (autoclave) or ethylene oxide gas (EOG) sterilization is performed. Furthermore, when the medicinal solution is filled, the sterilization treatment is performed using electron beams or ultraviolet rays before removal of the sealing member 82. When the sealing member 82 is removed, the inner sheet 3 is also removed together with the sealing member 82. This makes it possible to prevent the inner sheet 3 from being left in the container main body 20 when the medicinal solution is filled, leading to higher working efficiency.

Furthermore, similarly to the syringe package body 75 according to the sixth exemplary embodiment, since an outer edge of the inner sheet 3 is smaller than an inner edge of the opening portion 23 of the container main body 20, a gap is formed between the inner edge of the opening portion 23 of the container main body 20 and the outer edge of the inner sheet 3 (see Fig. 28). In addition, since the opposite region opposing to the sealing member 72 that is opposed to the inner sheet 3 includes a non-joint region where the sealing member 72 and the inner sheet 3 are not joined, a gap is generated also between the sealing member 72 and the inner sheet 3 (see Fig. 28). With formation of this gap, the steam or gas that has passed through the sealing member 72 at the time of sterilization easily enters below the inner sheet 3 in the container main body 20. This enables sterilization treatment using steam or gas to be efficiently performed onto the syringe 100 stored in the syringe storage container 1.

Since other configurations are similar to the configuration of the syringe package body 5 according to the first exemplary embodiment, description will be omitted. The above-configured syringe package body 85 including this sealing member 82 can also obtain the similar operational effects as the syringe package body 5 according to the first exemplary embodiment described above.

The exemplary embodiments of the present invention have been described as above including its operational effects. The syringe holding member and the syringe package body according to the present invention are not limited to the above-described embodiments, and various modifications are possible within the scope of the invention described in the claims.

While the above-described embodiment is an example in which two reinforcement ribs are provided between two predetermined adjacent holding tube portions, the present invention is not limited thereto, and three or more reinforcement ribs may be provided between the two predetermined adjacent holding tube portions.

With reference to Table 1 below, the number of holding tube portions, opening diameter of the cylindrical hole, and the ratio of the opening diameter of the cylindrical hole and the area of the base plate portion in the syringe holding member according to the above-described embodiment will be described. Note that the base plate portion of the syringe holding member has dimension settings of long side: a range of 220 mm to 240 mm and short side: a range of 190 mm to 210 mm. The thickness of the base plate portion has been set to a range of 0.8 mm to 2.5 mm.

**[Table 1]**

| NUMBER OF HOLDING TUBE PORTIONS | OPENING DIAMETER OF CYLINDRICAL HOLE OF HOLDING TUBE PORTION (mm) | RATIO OF TOTAL AREA OF OPENING TO AREA OF BASE PLATE PORTION (%) |
|---|---|---|
| 100 | 12.0 to 12.5 | 22 to 29 |
| 64 | 15.2 to 16.5 | 23 to 33 |
| 42 | 17.9 to 19.2 | 21 to 29 |

As illustrated in Table 1, a ratio of a total area of openings formed by the cylindrical holes of the holding tube portions penetrating through the base plate portion to an area of the upper surface of the base plate portion is in a range of 21% to 33%. For this reason, the base plate portion is more easily deformed by heat compared with a simple plate having no through hole. In contrast, as illustrated in the syringe holding members according to the above-described embodiments, two or more reinforcement ribs are provided between two predetermined holding tube portions adjacent to each other. This makes it possible to suppress deformation of the base plate portion which is easily deformed.

Furthermore, while the above-described embodiment is an example in which the first joint region for sealing the opening portion 23 of the container main body 20 with the sealing member 72 is formed by the first adhesive application region 72b coated with the adhesive, the first joint region may be formed by directly joining the container main body 20 and the sealing member 72 with each other by welding such as thermal welding or ultrasonic welding.

Note that the sealing member and the inner sheet described in the sixth and seventh exemplary embodiments described above can also be applied to packing bodies other than the above-described embodiment. More specifically, these sealing members and inner sheets may be applied to a syringe package body in which a plurality of reinforcement ribs connecting between adjacent holding tube portions are appropriately omitted from the syringe holding member. That is, these may be applied to a syringe package body including a syringe holding member including no reinforcement rib, including simply one reinforcement rib between each of a plurality of adjacent holding tube portions, or including a reinforcement rib merely at a portion between the plurality of adjacent holding tube portions.

Moreover, the syringe holding member itself may be omitted, and these may be applied to a syringe package body including a container main body including a plurality of holding portions for holding each of a plurality of syringes on the bottom portion of the container main body. Furthermore, these may be applied to a package body including a plurality of medicinal solution storage containers such as a plurality of vials and including a container main body including holding portions for holding the containers, instead of the plurality of syringes and syringe holding members.

Even in these cases, it is still possible to detach the inner sheet together with the sealing member when detaching the sealing member. Moreover, a gap is formed between the inner edge of the opening portion of the container main body and the outer edge of the inner sheet, and together with this, a gap is also formed between the sealing member and the inner sheet. With formation of these gaps, the steam or gas that has passed through the sealing member at the time of sterilization easily enters below the inner sheet in the container main body. In this manner, the sealing member and the inner sheet described in the sixth and seventh exemplary embodiments described above are effective in that it is possible to prevent the inner sheet from being left in the container main body to improve working efficiency, while it is possible to efficiently perform a sterilization treatment using steam or gas for the medicinal solution storage container stored in the container main body. Note that the medicinal solution storage container refers to a container such as a syringe or a vial including a filling port by which the medicinal solution is filled.

### Reference Signs List

1 Syringe storage container
2 Sealing member
5 Syringe package body
10, 30, 40, 50, 60 Syringe holding member
11, 31, 41, 51, 61 Base plate portion
11a, 31a, 41a, 51a, 61a Short side
11b, 31b, 41b, 51b, 61b Long side
12, 12A, 12C, 12B, 12D, 32, 32A, 32B, 32C, 32D, 42, 42A, 42B, 42C, 42D, 52, 52A, 52B, 52C, 52D, 62, 62A, 62A, 62B, 62C, 62D Holding tube portion
13, 33, 43, 53, 63 Reinforcement rib
20 Container main body
21 Bottom portion
22 Peripheral wall portion
23 Opening portion
24 Flange
25 Mounting shelf
100 Syringe
101 Syringe main body
102 Discharge portion
103 Filling port
104 Flange portion
106 Cap member

## Claims

1. A syringe holding member comprising:
a base plate portion formed in a substantially rectangular flat plate shape including a long side and a short side;
a plurality of holding tube portions protruding from an upper surface of the base plate portion, configured into which syringes are inserted, and including cylindrical holes that penetrate the base plate portion; and
a plurality of reinforcement ribs that protrudes from the upper surface of the base plate portion and connects each of the plurality of holding tube portions to each of the other plurality of adjacent holding tube portions,
wherein two or more of the reinforcement ribs are provided between two adjacent predetermined holding tube portions among the plurality of holding tube portions.

2. The syringe holding member according to claim 1,
wherein the plurality of holding tube portions is regularly arranged at equal intervals on the upper surface of the base plate portion, and
a plurality of the predetermined two holding tube portions is regularly arranged.

3. The syringe holding member according to claim 1 or 2,
wherein the plurality of holding tube portions is arranged in a plurality of rows in each of a first direction parallel to the long side of the base plate portion and a second direction parallel to the short side, and
at least one row among the plurality of rows is a reinforcement row in which the predetermined two holding tube portions are continuously arranged from one end to the other end in the at least one row of the plurality of rows.

4. The syringe holding member according to claim 3,
wherein a plurality of the reinforcement rows is provided in a direction different from a direction in which the predetermined two holding tube portions are continuously arranged among the first direction and the second direction.

5. The syringe holding member according to claim 3 or 4,
wherein the reinforcement row is provided at least in the first direction among the first direction and the second direction.

6. The syringe holding member according to any one of claims 3 to 5,
wherein each of the plurality of rows is the reinforcement row.

7. The syringe holding member according to any one of claims 1 to 6,
wherein the base plate portion includes the long side being a range of 220 mm to 240 mm, the short side being a range of 190 mm to 210 mm, and a thickness of the base plate portion being a range of 0.8 mm to 2.5 mm.

8. The syringe holding member according to claim 7,
wherein a ratio of a total area of openings formed by the cylindrical holes of the plurality of holding tube portions penetrating through the base plate portion to an area of the upper surface of the base plate portion has been set to a range of 21% to 33%.

9. A syringe package body comprising:
the syringe holding member according to any one of claims 1 to 8;
a plurality of syringes held by each of the plurality of holding tube portions of the syringe holding member;
a container main body including: a peripheral wall portion including an upper end portion and a lower end portion; a bottom portion continuous with the lower end portion of the peripheral wall portion, the bottom portion being surrounded by the lower end portion; an opening portion surrounded by the upper end portion; a storage space formed by the peripheral wall portion, the bottom portion, and the opening portion; and a mounting shelf formed in the peripheral wall portion; and
a sheet-like sealing member that seals the opening portion of the container main body and is formed of a material including gas permeability and bacteria impermeability,
wherein the syringe holding member has been mounted on the mounting shelf and stored in the storage space.

10. The syringe package body according to claim 9, the syringe package body further comprising an inner sheet disposed between the sealing member and the plurality of syringes held by the syringe holding member to cover each of the plurality of syringes and including an outer edge smaller than an inner edge of the opening portion of the container main body,
wherein the sealing member includes:
an opposite region opposing to the inner sheet, and
a first joint region, in which the sealing member and the container main body are joined, closer to a peripheral edge side of the sealing member than the opposite region, and
the opposite region includes:
a second joint region in which the sealing member and the inner sheet are joined; and
a non-joint region in which the sealing member and the inner sheet are not joined.

11. A method for assembling a syringe package body, the method comprising:
preparing a syringe holding member comprising: a base plate portion formed in a substantially rectangular flat plate shape including a long side and a short side; a plurality of holding tube portions which protrudes from an upper surface of the base plate portion, into which syringes are inserted, and which includes a cylindrical holes that penetrate the base plate portion; and a plurality of reinforcement ribs protruding from the upper surface of the base plate portion and connecting each of the plurality of holding tube portions to each of the other plurality of adjacent holding tube portions, two or more of the reinforcement ribs being provided in the syringe holding member between two predetermined adjacent holding tube portions among the plurality of holding tube portions,
preparing a container main body including: a peripheral wall portion including an upper end portion and a lower end portion; a bottom portion continuous with the lower end portion of the peripheral wall portion, the bottom portion being surrounded by the lower end portion; an opening portion surrounded by the upper end portion; a storage space formed by the peripheral wall portion, the bottom portion, and the opening portion; and a mounting shelf formed in the peripheral wall portion;
inserting each of a plurality of syringes into each of the plurality of holding tube portions of the syringe holding member;
mounting the syringe holding member, which holds the plurality of syringes, on the mounting shelf of the container main body so as to store the syringe holding member in the storage space;
sealing the opening portion of the container main body by a sheet-like sealing member formed of a material including gas permeability and bacteria impermeability; and
sterilizing the plurality of syringes, the syringe holding member, the container main body, and the sealing member using high pressure steam.

12. The method for assembling a syringe package body according to claim 11, further comprising, before the sealing by the sealing member,
arranging an inner sheet configured to cover each of the plurality of syringes and including an outer edge smaller than an inner edge of the opening portion of the container main body on the plurality of syringes held by the syringe holding member,
wherein, during the sterilizing using the high pressure steam, the sealing member is recessed to an inner side of the container main body, and the sealing member and the inner sheet are bonded with each other by a thermoplastic adhesive provided on the sealing member.
